# EUROPÄISCHE PATENTSCHRIFT

(11) **EP 2 953 669 B1**
(45) Veröffentlichungstag und Bekanntmachung des Hinweises auf die Patenterteilung: **24.10.2018**
(21) Anmeldenummer: 13703047.4
(22) Anmeldetag: 06.02.2013
(51) Int. Cl.: A61M 5/315, A61M 5/31, A61M 5/32

(54) **ABGABEVORRICHTUNG ZUR ABGABE EINES FLUIDS**
DISPENSING DEVICE FOR DISPENSING A FLUID
DISPOSITIF DE DISTRIBUTION PERMETTANT DE DISTRIBUER UN FLUIDE

(43) Veröffentlichungstag der Anmeldung: 16.12.2015
(73) Patentinhaber: Weibel CDS AG, 9104 Waldstatt (CH)
(72) Erfinder: WEIBEL, Ludwig Daniel, CH-9104 Waldstatt (CH)
(74) Vertreter: Hepp Wenger Ryffel AG
(86) Internationale Anmeldenummer: PCT/EP2013/052296
(87) Internationale Veröffentlichungsnummer: WO 2014/121822

(56) Entgegenhaltungen:
- EP-A1- 2 752 211
- WO-A1-2007/144507
- WO-A1-2012/137803
- WO-A1-2012/138318
- WO-A2-2005/105014
- US-A- 1 831 668
- US-A- 3 747 812
- US-A- 3 989 045
- US-A1- 2008 300 549

## Beschreibung

Die Erfindung betrifft eine Abgabevorrichtung zur Abgabe eines Fluids, insbesondere zur medizinischen Anwendung, vorzugsweise eine Injektionsspritze, gemäss dem Oberbegriff von Anspruch 1.

Abgabevorrichtungen zur Abgabe eines Fluids umfassen z.B. Infusionsvorrichtungen, Injektionsspritzen sowie Instillationsspritzen. Derartige Abgabevorrichtungen sollen nur zum einmaligen Gebrauch geeignet sein und eine hohe Manipulationssicherheit aufweisen. Solche Vorrichtungen können selbstverständlich auch im nichtmedizinischen Bereich Anwendung finden, beispielsweise für chemische Analysezwecke im Lebensmittelbereich oder im Kosmetikbereich.

Die WO 2007/144507 A1 beschreibt eine geschützte Vorrichtung zur Injektion eines medikamentösen Produktes mit einem Spritzenkörper, der eine Injektionsnadel trägt und mit einem Kolben versehen ist. Die Vorrichtung umfasst eine Schutzhülle, die entlang dem Spritzenkörper in eine Schutzposition gleiten kann, in welcher die Injektionsnadel vollständig in der Schutzhülle untergebracht ist. Dadurch kann verhindert werden, dass sich ein Benutzer nach Gebrauch der Vorrichtung an der Injektionsnadel verletzt. Die WO 2012/137803 A1 beschreibt ein Injektionsgerät mit einer inneren Struktur bestehend aus Zylinder, Injektionsnadel und Kolben und mit einer äusseren zylindrischen Schutzhülle. Die EP 2 752 211 A1 betrifft eine Sicherheitsvorrichtung an einer Spritze. Ausserhalb des Spritzenzylinders ist eine Schutzhülle unter Federvorspannung gehalten, welche zur Überdeckung der Nadel ausgelöst werden kann.

Es ist daher die Aufgabe der Erfindung, die Nachteile des Standes der Technik zu überwinden. Insbesondere ist es eine Aufgabe der Erfindung, eine einfach und sicher handhabbare Abgabevorrichtung, insbesondere eine Injektionsspritze, bevorzugt zum einmaligen Gebrauch, bereitzustellen. Die Abgabevorrichtung soll zudem vielseitig anwendbar sein, insbesondere auch in Verbindung mit einem Transfersystem zum Transfer eines Fluids.

Eine Abgabevorrichtung zur Abgabe eines Fluids, insbesondere zur medizinischen Anwendung, vorzugsweise eine Injektionsspritze umfasst einen Grundkörper mit einer Längsachse und einen im Grundkörper ausgebildeten Aufnahmeraum für das Fluid. Die Abgabevorrichtung umfasst weiter einen Abgabebereich, welcher zur Anordnung einer mit dem Aufnahmeraum kommunizierende Kanüle ausgebildet ist oder in welchem eine mit dem Aufnahmeraum kommunizierende Kanüle angeordnet ist, durch welche Kanüle das Fluid aus dem Aufnahmeraum abgebbar ist. Die Kanüle ist insbesondere eine Injektionsnadel.

Dabei umfasst die Abgabevorrichtung eine Schutzvorrichtung mit einem am Grundkörper angeordneten starren Schutzkörper. Die Schutzvorrichtung ist bevorzugt zum Abdecken einer Kanüle nach der Abgabe eines Fluids aus der Abgabevorrichtung ausgebildet. Der Schutzkörper ist derart ausgebildet und bezüglich dem Grundkörper derart zwischen einer Bereitschaftsstellung und einer Schutzstellung beweglich, dass die im Abgabebereich angeordnete oder anordenbare Kanüle in der Bereitschaftsstellung vollständig freigelegt ist und sich der Schutzkörper in der Schutzstellung vom Grundkörper vollständig über die im Abgabebereich angeordnete oder anordenbare Kanüle hinaus erstreckt.

Typischerweise ist eine im Abgabebereich anordenbare Kanüle in der Bereitschaftsstellung vollständig freigelegt, wenn eine Kopplungsstelle zum Ankoppeln der Kanüle zumindest teilweise vom Schutzkörper freigegeben ist. Da derartige Kanülen oft genormte Längen haben, erschliesst sich unmittelbar, wie weit der Schutzkörper sich in der Schutzstellung über den Grundkörper hinaus erstrecken muss, um vollständig über die Kanüle hinauszuragen, auch wenn diese nicht fest an der Abgabevorrichtung angeordnet ist.

Typischerweise können je nach Anwendung Kanülen mit Längen im Bereich von etwa 10 mm bis 120 mm zur Anwendung kommen. Beispielsweise sind im Falle von Insulininjektionen vergleichsweise kurze Kanülen mit Längen von etwa 10 mm bis 20 mm typisch, während z.B. im Dentalbereich Längen von etwa 10 mm bis 45 mm zur Anwendung kommen. Bei intramuskulären, subkutanen oder intravenösen bzw. intraarteriellen Injektionen liegen die Längen der Kanülen typischerweise im Bereich von 20 mm bis 80 mm. Im Bereich der Neuraltherapie werden sogar Kanülen mit Längen von bis zu 120 mm eingesetzt. Je nachdem mit welchen Kanülen die Abgabevorrichtung zur Anwendung kommen soll, erstreckt sich der Schutzkörper in der Schutzstellung derart weit über den Grundkörper hinaus, dass er vollständig über die zur Anwendung vorgesehenen Kanülen hinausragen kann.

Dabei ist ein von einem Benutzer direkt oder indirekt auslösbares Auslösemittel vorhanden, mit welchem ein Übergang des Schutzkörpers aus der Bereitschaftsstellung in die Schutzstellung auslösbar ist. Dabei ist ein Sicherungsmittel vorhanden, mit welchem die Schutzvorrichtung in einen gesicherten und einen ungesicherten Zustand bringbar ist, wobei die Schutzvorrichtung im gesicherten Zustand gegen eine Auslösung des Übergangs von der Bereitschaftsstellung in die Schutzstellung gesichert ist, und im ungesicherten Zustand der Übergang auslösbar ist.

Der Schutzkörper kann dabei z.B. U-förmig, rohrförmig oder hülsenförmig ausgebildet sein, sodass die Kanüle in der Schutzstellung in einem zumindest teilweise vom Schutzkörper umschlossenen Innenraum des Schutzkörpers angeordnet ist. Auf diese Weise ist die Kanüle geschützt, sodass sich ein Benutzer nicht mehr an der Kanüle verletzen kann. Der Schutzkörper kann dabei derart ausgebildet sein, dass in der Bereitschaftsstellung der Grundkörper zumindest teilweise vom Schutzkörper umschlossen ist. Bevorzugt ist der Schutzkörper daher als Hülse ausgebildet, welche in Längsrichtung des Grundkörpers bei einem Übergang in die Schutzstellung über den Abgabebereich und die Kanüle hinaus verschiebbar ist.

Der Schutzkörper ist bezüglich dem Grundkörper bevorzugt mit einer Federkraft derart beaufschlagt, dass beim Auslösen der Auslösemittel automatisch der Übergang in die Bereitschaftsstellung erfolgt. Hierzu ist, bevorzugt im Abgabebereich, eine Feder vorgesehen, welche zwischen dem Grundkörper und der Schutzvorrichtung wirkt und den Schutzkörper mit einer Federkraft in Richtung zur Schutzstellung beaufschlagt.

Bevorzugt umfasst die Schutzvorrichtung Arretierungsmittel, welche den Schutzkörper in der Schutzstellung am Grundkörper arretieren, wenn diese einmal eingenommen wurde, sodass die Abgabevorrichtung nicht mehr benutzt werden kann. Hierzu kann der Schutzkörper z.B. Rastklinken umfassen, welche mit einer Rastkante des Grundkörpers in der Schutzstellung derart zusammenwirken, dass der Schutzkörper nicht mehr in die Bereitschaftsstellung gebracht werden kann.

Das Auslösemittel kann z.B. ein auslösbares Arretierungsmittel umfassen, welches bei der Benutzung der Abgabevorrichtung bei der Abgabe des Fluids auslösbar ist. Das Auslösemittel ist dabei zu unterscheiden von dem Sicherungsmittel, welches den Schutzkörper im gesicherten Zustand fest arretiert und gegen einen Übergang in die Schutzstellung sichert. Das Auslösemittel ist gemäss diesem Aspekt der Erfindung nur im ungesicherten Zustand auslösbar.

Das Sicherungsmittel ist insbesondere bei Abgabevorrichtungen vorteilhaft, welche erst kurz vor der Benutzung mit einem Fluid befüllt werden, d.h. z.B. aufgezogen werden. Durch das Sicherungsmittel ist sichergestellt, dass die Schutzvorrichtung nicht versehentlich ausgelöst werden kann, bevor die Abgabevorrichtung überhaupt in einem abgabebereiten Zustand ist. Bevorzugt ist das Sicherungsmittel derart ausgebildet, dass die Schutzvorrichtung beim oder nach dem Befüllen, d.h. insbesondere sobald sie in einem abgabebereiten Zustand ist, automatisch im ungesicherten Zustand ist.

Das Sicherungsmittel ist derart ausgebildet, dass im gesicherten Zustand der Schutzvorrichtung das Auslösemittel vom Sicherungsmittel gegen eine Auslösung gesichert ist. Indem das Sicherungsmittel das Auslösemittel gegen eine Auslösung sichert, ist auf einfache Weise sichergestellt, dass keine Auslösung erfolgen kann. Bevorzugt wirkt das Sicherungsmittel entgegen einer Auslöserichtung auf das Auslöselement. In Varianten kann das Sicherungsmittel auch auf den Schutzkörper wirken und diesen arretieren, sodass die Auslösemittel auslösbar sind, der Schutzkörper aber nicht in die Schutzstellung bewegt werden kann.

Das Auslösemittel umfasst wenigstens eine Rastzunge, welche am Schutzkörper ausgebildet ist und in der Bereitschaftsstellung des Schutzkörpers an einer Rastkante des Grundkörpers verrastet ist. Die Rastkante kann dabei an einem dem Abgabebereich gegenüberliegenden Längsende des Grundkörpers ausgebildet sein. Bevorzugt umgreift die Rastzunge mit einem Rastvorsprung die Rastkante derart, z.B. von aussen her, dass eine Auslösung durch einfaches Wegbiegen der Rastzunge vom Grundkörper erfolgen kann.

Die Abgabevorrichtung weist eine Betätigungsvorrichtung mit einem beweglichen Kolben im Aufnahmeraum auf, welcher zum Einbringen des Fluids in den Aufnahmeraum aus einer eingeschobenen Stellung in eine ausgezogene Stellung und zum Ausbringen in umgekehrter Richtung beweglich ist. Der Aufnahmeraum sowie der Kolben sind dabei typischerweise einander entsprechend kreiszylindrisch ausgebildet, wobei der Kolben allenfalls mit einer radial wirkenden Dichtung versehen ist, um einen für die Verschiebbarkeit vorhandenen Toleranzspalt zwischen Kolben und Innenwand des Aufnahmeraums abzudichten.

Die Betätigungsvorrichtung kann eine Kolbenstange umfassen, welche an einem dem Abgabebereich gegenüberliegenden, offenen Längsende des Grundkörpers aus dem Aufnahmeraum nach aussen ragt und beispielsweise eine Fingerplatte zum Verschieben des Kolbens durch einen Benutzer aufweist. Durch Zug an der Fingerplatte in Längsrichtung kann die Abgabevorrichtung aufgezogen werden, d.h. kann der Kolben in eine ausgezogene Stellung gebracht werden. Durch Druck auf die Fingerplatte in Längsrichtung kann das Fluid durch die Abgabeöffnung nach aussen gepresst werden, wobei der Kolben in die eingeschobene Stellung bringbar ist.

Vorliegend sind als "ausgezogene Stellung" verschiedene Stellungen bezeichnet, bei welchen der Kolben aus der eingeschobenen Stellung von der Abgabeöffnung weg bewegt ist. Als eingeschobene Stellung ist hingegen eine Stellung bezeichnet, in welcher der Kolben im Wesentlichen vollständig im Aufnahmeraum zur Abgabeöffnung hin verschoben ist.

Dabei ist die Betätigungsvorrichtung bzw. ein Teil der Betätigungsvorrichtung derart als Sicherungsmittel ausgebildet, das die Schutzvorrichtung in der eingeschobenen Stellung des Kolbens im gesicherten Zustand sein kann. Auf diese Weise kann die Schutzvorrichtung in einem gesicherten Zustand sein, wenn die Abgabevorrichtung, insbesondere vor einer Benutzung, kein Fluid enthält bzw. noch nicht aufgezogen wurde.

Bevorzugt ist die Betätigungsvorrichtung derart ausgebildet, dass beim Übergang aus der ausgezogenen Stellung in die eingeschobene Stellung des Kolbens das Auslösemittel, insbesondere zwingend, auslösbar ist. Auf diese Weise ist sichergestellt, dass bei der Abgabe des Fluids, d.h. beispielsweise bei der Durchführung einer Injektion, automatisch ein Übergang der Schutzvorrichtung in die Schutzstellung auslösbar ist.

Mit Vorteil ist das Sicherungsmittel derart ausgebildet, dass in einer ausgezogenen Stellung des Kolbens die Schutzvorrichtung, insbesondere zwingend, im ungesicherten Zustand ist. D.h. sobald die Abgabevorrichtung ganz oder teilweise aufgezogen ist, ist die Schutzvorrichtung in einem ungesicherten Zustand. Auf diese Weise ist sichergestellt, dass, sobald die Abgabevorrichtung in einem abgabebereiten Zustand ist, die Schutzvorrichtung in einem ungesicherten Zustand ist und ausgelöst werden kann. Das Sicherungsmittel ist bevorzugt derart ausgebildet, dass nach einem Übergang vom gesicherten in den ungesicherten Zustand der Schutzvorrichtung der gesicherte Zustand nicht mehr herstellbar ist.
Die Betätigungsvorrichtung weist eine, insbesondere starr mit dem Kolben verbundene, Fingerplatte zum Verschieben des Kolbens auf, welche als Sicherungsmittel ausgebildet ist. Indem die Fingerplatte als Sicherungsmittel ausgebildet ist, kann die Abgabevorrichtung besonders einfach und ohne zusätzliche Teile ausgebildet sein. Beim Verschieben des Kolbens in eine ausgezogene Stellung wird die Fingerplatte vom Auslösemittel wegbewegt, womit eine Wirkverbindung des Sicherungsmittels mit dem Auslösemittel unterbrochen wird, sodass dieses in einen ungesicherten Zustand übergeht. Der Übergang ist bevorzugt irreversibel, sodass der gesicherte Zustand nicht wieder herstellbar ist (siehe unten).

Das Sicherungsmittel ist als wenigstens ein Durchbruch in der Fingerplatte ausgebildet, durch welchen das Auslösemittel, insbesondere gegebenenfalls die wenigstens eine Rastzunge, hindurchragen kann, wenn der Kolben in der eingeschobenen Stellung ist. Der Durchbruch ist dabei derart ausgebildet, dass die Rastzunge gegen eine Auslösung gesperrt ist, solange sie durch den Durchbruch hindurchragt.

Der Durchbruch weist hierzu einen Anschlag auf, welcher das Auslösemittel in einer Auslöserichtung sperrt. Der Anschlag ist bezüglich der Rastzunge sowie der Rastkante des Grundkörpers derart angeordnet, dass die Rastzunge vom Anschlag an der Rastkante festlegbar ist. Auf diese Weise bildet der Anschlag des Durchbruchs bezüglich der Rastkante ein Gegenlager für die Rastzunge, welches ein Auslösen verhindert. Mit anderen Worten legt der Anschlag des Durchbruchs das Auslösemittel, insbesondere die Rastzunge, in dessen Auslöserichtung, insbesondere bezüglich der Rastkante, derart fest, dass eine Auslösung nicht möglich ist.

Bevorzugt weist das Sicherungsmittel und/oder das Auslösemittel ein Sperrmittel auf, welches derart ausgebildet ist, dass ein Wiederherstellen der Wirkverbindung, insbesondere zwischen Sicherungsmittel und Auslösemittel, des gesicherten Zustand verhindert ist, wenn diese Wirkverbindung einmal gelöst wurde.

Das Sperrmittel kann insbesondere im Falle einer Rastzunge bevorzugt derart ausgebildet sein, dass die Rastzunge zwar in einer Richtung durch den Durchbruch der Fingerplatte ausbringbar ist, in Gegenrichtung aber gegen ein Einbringen in den Durchbruch gesperrt ist. Hierzu weist die Rastzunge bevorzugt an einem in gesichertem Zustand durch den Durchbruch hindurchragenden Bereich ein Sperrmittel auf, welches als, vorzugsweise deformierbare, Aufweitung ausgebildet ist. Die Aufweitung erstreckt sich dabei bevorzugt in einer Projektion längs der Bewegungsrichtung der Fingerplatte über einen lichten Querschnitt des Durchbruchs hinaus.

Die Aufweitung kann aus dem Durchbruch ausgebracht werden, indem die Aufweitung z.B. deformiert wird. Ebenso kann die Aufweitung derart ausgelenkt werden, dass sie in einen Bereich des Durchbruchs bringbar ist, in welchem sie nicht mit dem Durchbruch überlappt und durch diesen durchführbar ist.

Nachdem die Rastzunge aus dem Durchbruch ausgebracht ist, kann die Aufweitung gegebenenfalls z.B. aufgrund elastischer Deformation wieder in die ursprünglich überlappende Form zurückkehren. Bei einer Auslenkung zum Ausbringen kann die Rastzunge z.B. wieder in die ursprüngliche Position zurückgestellt werden, d.h. in einen Bereich des Durchbruchs, in welchem die Aufweitung mit diesem überlappt (z.B. automatisch aufgrund einer federelastischen Wirkung der Rastzunge). Aufgrund der Aufweitung kann die Rastzunge somit nicht wieder in den Durchbruch gelangen, wenn die Fingerplatte bzw. der Durchbruch in die entgegengesetzte Richtung, d.h. bevorzugt zum Grundkörper hin verschoben wird.

Die Aufweitung kann zum freien Ende der Rastzunge hin z.B. auseinander laufend ausgebildet sein, um ein einfaches Ausbringen aus dem Durchbruch in die eine Richtung und ein Sperren gegen das Einbringen in der anderen Richtung sicherzustellen. Die Aufweitung kann hierzu z.B. in die Sperrrichtung spreizbar ausgebildet sein. An der Aufweitung kann beispielsweise ein Deformationsspalt vorhanden sein, sodass vom Deformationsspalt getrennte Abschnitte beim Ausbringen aus dem Durchbruch zusammengedrückt werden können. In Gegenrichtung sind die beiden Abschnitte aufgrund des Deformationsspalts hingegen zum Sperren spreizbar.

Grundsätzlich ist es in Varianten auch denkbar, das Auslösemittel mit einer Garantielasche mit einer Sollbruchstelle zu versehen, welche beim Übergang vom gesicherten in den ungesicherten Zustand abreissbar ist. Die fehlende Garantielasche verhindert in diesem Fall bevorzugt, dass das Sicherungsmittel mit dem Auslösemittel zur Wiederherstellung des gesicherten Zustands zusammenwirken kann. Dies kann z.B. auf einfache Weise dadurch erreicht werden, dass das Sicherungsmittel nur über die Garantielasche mit dem Auslösemittel zusammenwirkt. Es versteht sich, dass diese Ausführungsform nicht auf eine Ausbildung des Auslösemittels als Rastzunge beschränkt ist sondern auch allgemein bei anderen Ausgestaltungen des Auslösemittels vorteilhaft sein kann.

Bevorzugt weist die Betätigungsvorrichtung eine, vorzugsweise an der Fingerplatte, insbesondere an einer dem Grundkörper zugewandten Seite, ausgebildete, Steuerfläche auf, welche beim Übergang aus einer ausgezogenen Stellung in die eingeschobene Stellung des Kolbens derart mit dem Auslösemittel, insbesondere die Rastzunge, zusammenwirkt, dass das Auslösemittel von der Steuerfläche in eine Auslöserichtung bewegbar ist bzw. bewegt wird, wobei insbesondere eine Verrastung der Rastzunge an der Rastkante des Grundkörpers lösbar ist.

Umgreift die Rastzunge in der Bereitschaftsstellung der Schutzvorrichtung beispielsweise einen Rand des Grundkörpers, kann die Steuerfläche z.B. von einer Kolbenstange zur Fingerplatte hin auseinander laufender ausgebildet sein. Auf diese Weise wird die Rastzunge nach aussen gezwungen, wenn die Fingerplatte zum Grundkörper hin bewegt wird. Es erschliesst sich unmittelbar, wie eine Auslösung des Auslösemittels auch bei anderer Ausbildung und Anordnung erfolgen kann, indem eine Bewegung der Fingerplatte bzw. anderer Teile der Betätigungsvorrichtung in eine Bewegung des Auslösemittels in Auslösrichtung umgesetzt wird.

Es versteht sich, dass auch mehrere Auslösemittel, insbesondere mehrere Rastzungen, vorhanden sein können. Entsprechend können auch mehrere Sicherungsmittel, insbesondere Durchbrüche, an der Fingerplatte, vorhanden sein, welche im gesicherten Zustand jedes Auslösemittel gegen eine Auslösung sichern. Gegebenenfalls ist es bei mehreren Auslösemitteln aber auch ausreichend, nur eines der Auslösemittel gegen eine Auslösung zu sichern.

Die Erfindung wird im Folgenden anhand von Abbildungen von exemplarisch dargestellten Ausführungsbeispielen näher erläutert. Es zeigen schematisch:
- Fig. 1:: eine Seitenansicht eines Grundkörpers einer erfindungsgemässen Abgabevorrichtung;
- Fig. 2:: eine weitere Seitenansicht des Grundkörpers gemäss Fig. 1;
- Fig. 3:: eine Schrägansicht des Grundkörpers gemäss Fig. 1;
- Fig. 4:: eine Ausschnittsansicht eines Abgabebereichs des Grundkörpers der Fig. 1;
- Fig. 5:: eine weitere Ausschnittsansicht des Abgabebereichs des Grundkörpers der Fig. 1;
- Fig. 6:: eine Seitenansicht eines Verschlusselements für eine erfindungsgemässe Abgabevorrichtung;
- Fig. 7:: eine weitere Seitenansicht des Verschlusselements gemäss Fig. 6;
- Fig. 8:: eine Schrägansicht des Verschlusselements gemäss Fig. 6;
- Fig. 9:: eine Draufsicht auf das Verschlusselement der Fig. 6 längs einer Längsachse;
- Fig. 10:: eine weitere Draufsicht auf das Verschlusselement der Fig. 6 längs einer Längsachse entgegen der Blickrichtung der Fig. 9;
- Fig. 11:: eine Seitenansicht der Abgabevorrichtung mit dem im Abgabebereich angeordneten Verschlusselement;
- Fig. 12:: eine Schutzkappe für eine Abgabevorrichtung mit einem Transferkanal zur Zuführung eines Fluids;
- Fig. 13:: eine Ausschnittsansicht eines Sitzes der Schutzkappe der Fig. 12 für ein Kopplungsmittel der Abgabevorrichtung;
- Fig. 14:: eine Ausschnittsansicht des mit der Schutzkappe versehenen Abgabebereichs der Abgabevorrichtung mit dem Verschlusselement in einer Füllstellung;
- Fig. 15:: eine Teilansicht eines Querschnitts der Ansicht der Fig. 14;
- Fig. 16:: eine Querschnittsansicht der Ansicht der Fig. 14 in einer Ebene senkrecht zur Ansicht der Fig. 15;
- Fig. 17:: die Ausschnittsansicht gemäss Fig. 14, wobei das Verschlusselement in einer Abgabestellung ist, kurz vor dem Ausbringen aus der Schutzkappe;
- Fig. 18:: eine Teilansicht eines Querschnitts der Ansicht der Fig. 17;
- Fig. 19:: eine Querschnittsansicht der Ansicht der Fig. 17 in einer Ebene senkrecht zur Ansicht der Fig. 18;
- Fig. 20:: eine Seitenansicht eines Schutzkörpers einer Schutzvorrichtung;
- Fig. 21:: eine weitere Seitenansicht des Schutzkörpers der Fig. 20;
- Fig. 22:: eine Ausschnittsansicht von Auslösemitteln des Schutzkörpers der Fig. 20;
- Fig. 23:: eine Aussenansicht einer Abgabevorrichtung mit einem Grundkörper und einer Schutzvorrichtung in einem gesicherten Zustand;
- Fig. 24:: eine Querschnittsansicht der Ansicht der Fig. 23;
- Fig. 25:: eine Ausschnittsansicht der Ansicht der Fig. 24 im Bereich der Auslösemittel der Schutzvorrichtung;
- Fig. 26:: eine Querschnittsansicht in einer Schnittebene gemäss Fig. 25;
- Fig. 27:: eine Aussenansicht der Abgabevorrichtung der Fig. 23 in einem ungesicherten Zustand;
- Fig. 28:: eine Teilansicht eines Querschnitts der Ansicht der Fig. 27;
- Fig. 29:: eine Querschnittsansicht in einer Schnittebene gemäss Fig. 28;
- Fig. 30:: eine Aussenansicht der Abgabevorrichtung der Fig. 23 im Moment der Auslösung der Schutzvorrichtung;
- Fig. 31:: eine Teilansicht eines Querschnitts der Ansicht der Fig. 30 im Bereich der Auslösemittel;
- Fig. 32:: eine Querschnittsansicht in einer Schnittebene gemäss Fig. 32;
- Fig. 33:: eine Ausschnittsansicht der Ansicht der Fig. 32;
- Fig. 34:: eine Aussenansicht der Abgabevorrichtung der Fig. 24, wobei die Schutzvorrichtung in einer Schutzstellung ist;
- Fig. 35:: eine Aussenansicht einer weiteren Ausführungsform eines Grundkörpers einer erfindungsgemässen Abgabevorrichtung mit einer Schutzvorrichtung.

Grundsätzlich sind einander entsprechende Elemente mit denselben Bezugszeichen versehen.

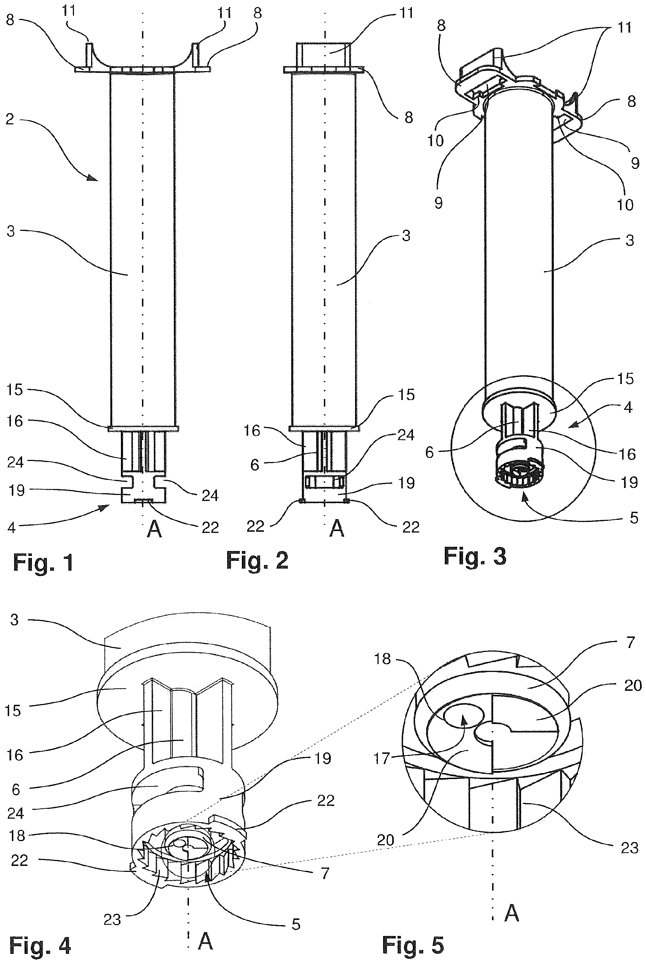

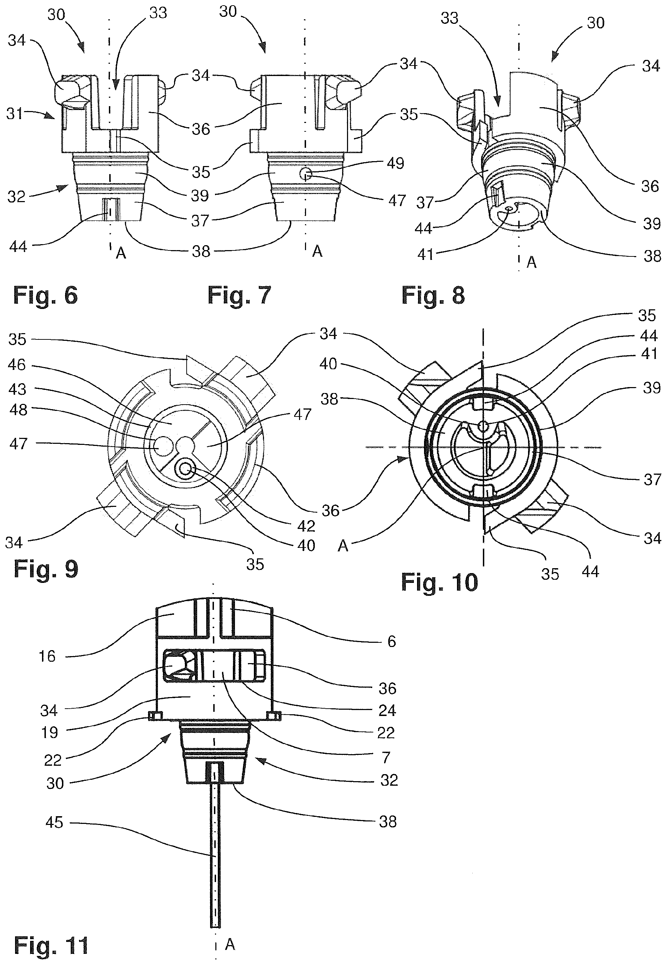

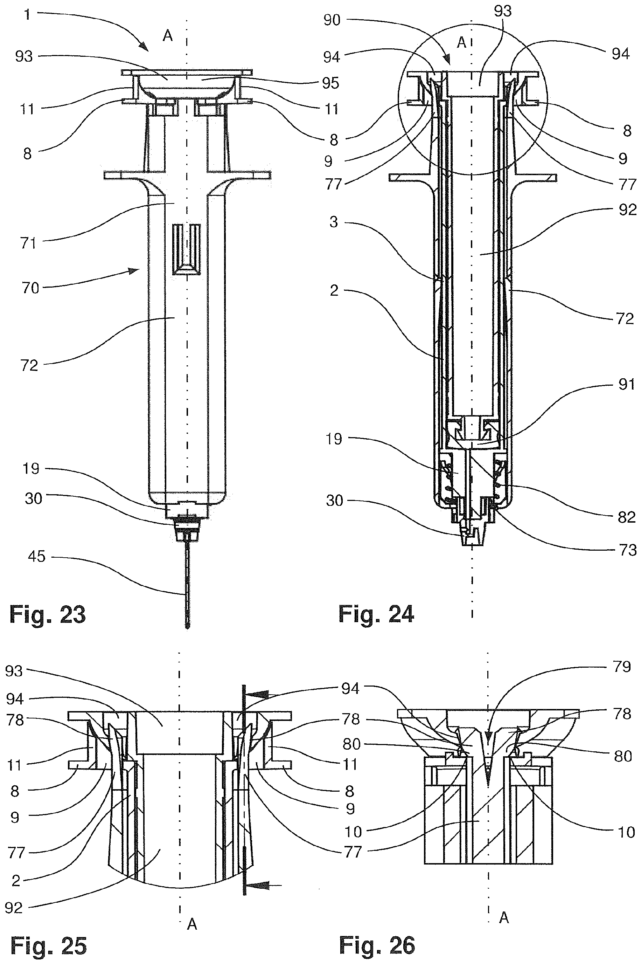

Figur 1 zeigt eine Seitenansicht eines Grundkörpers 2 einer erfindungsgemässen Abgabevorrichtung 1 (siehe z.B. Fig. 23). Figur 2 zeigt eine weitere Seitenansicht des Grundkörpers 2 in einer Ansicht senkrecht zur Blickrichtung der Fig. 1. Figur 3 zeigt eine äussere Schrägansicht des Grundkörpers 2. Figur 4 zeigt eine Ausschnittsansicht in einem Abgabebereich 4 des Grundkörpers 2. Figur 5 zeigt eine Ausschnittsansicht eines Aufnahmeraums 5 für ein Verschlusselement 30 (siehe z.B. Fig. 6 - 10) mit einer Abgabeöffnung 18 des Grundkörpers 2. Die Fig. 1 bis 5 sind im Folgenden gemeinsam beschrieben.

Der Grundkörper 2 weist einen kreiszylindrischen, rohrförmigen Abschnitt 3 mit einer Längsachse A auf. Der Abgabebereich 4 ist an einem vorderen Längsende des Grundkörpers 2 ausgebildet. Im Folgenden wird eine Richtung längs A zum Abgabebereich 4 hin mit "vorne" bezeichnet, während eine entgegengesetzte Richtung mit "hinten" bezeichnet ist.

An einem hinteren Längsende des Grundkörpers 2 sind auf gegenüberliegenden Seiten in Richtung senkrecht zu A auskrangende Flügel 8 ausgebildet. Die Flügel 8 weisen jeweils in Richtung von A Durchbrüche 9 auf, in welchen Rastkanten 10 für Rastzungen 77 einer Schutzvorrichtung 70 ausgebildet sind. In Richtung von A nach hinten ragend sind Stützstrukturen 11 ausgebildet, welche einerseits die Stabilität der Flügel 8 verbessern und zum anderen Anschläge für eine Fingerplatte 93 einer Betätigungsvorrichtung 90 der Abgabevorrichtung 1 bilden (siehe z.B. Fig. 23). Zudem dienen die Stützstrukturen 11 zum Schutz der Rastzungen 77, welche in einer Bereitschaftsstellung der Schutzvorrichtung 70 durch die Durchbrüche 9 nach hinten ragen (siehe hierzu z.B. Fig. 24 - 27).

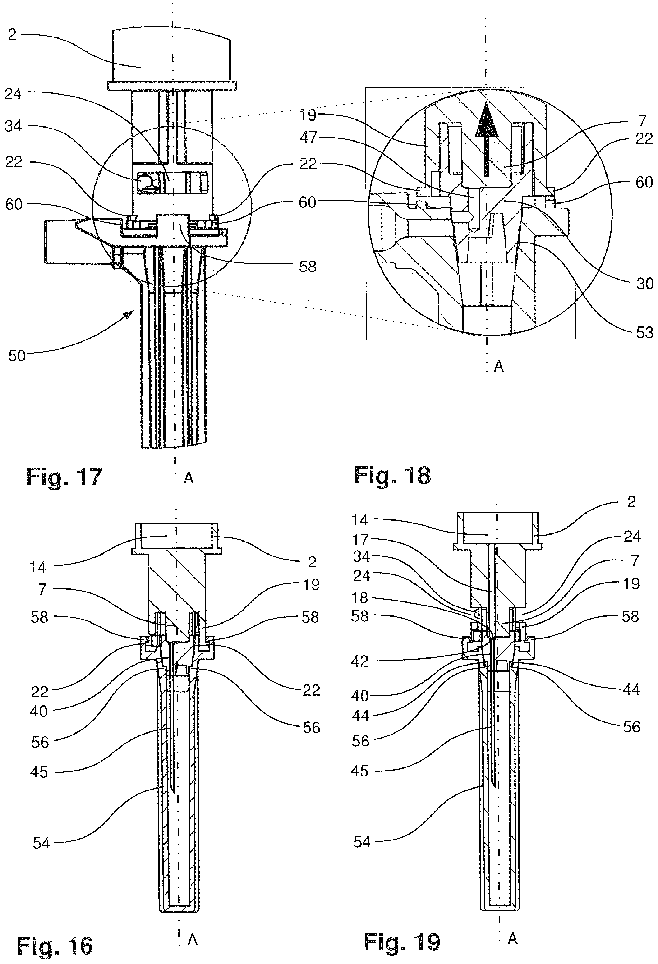

Im zylindrischen Abschnitt 3 ist ein Aufnahmeraum 14 zur Aufnahme eines Fluids ausgebildet (siehe z.B. Fig. 16 und 19). Der Aufnahmeraum 14 weist einen kreiszylindrischen Querschnitt auf und ist am hinteren Längsende des Grundkörpers 2 offen. Am vorderen Längsende ist der Aufnahmeraum 14 von einem deckelförmigen Abschluss 15 verschlossen, vor welchem der Abgabebereich 4 angeordnet ist. Ausgehend vom Abschluss 15 erstreckt sich in Längsrichtung A eine Trägerstruktur mit kreuzförmigen Rippen 16 nach vorne. Längs der Kreuzungslinie der Rippen 16 ist eine Verdickung 6 ausgebildet, in welcher sich ein Fluidkanal 17 (siehe Fig. 19) in Längsrichtung A zum Aufnahmeraum 5 für das Verschlusselement 30 erstreckt. Die Verdickung 6 ist weitgehend kreiszylindrisch in Richtung von A ausgebildet und exzentrisch bezüglich der Längsachse A versetzt. Der Fluidkanal 17 kommuniziert mit dem Aufnahmeraum 14.

Der Aufnahmeraum 5 ist von einem Innenraum eines weitgehend kreiszylindrischen Rohrabschnitts 19 gebildet, welcher in Richtung nach vorne an die Rippen 16 anschliesst und koaxial mit der Längsachse A angeordnet ist. Der Aufnahmeraum 5 ist nach vorne im Wesentlichen über seinen gesamten Querschnitt offen. Im Aufnahmeraum 5 ist koaxial mit A ein Lagerzapfen 7 für das Verschlusselement 30 angeordnet. Am Lagerzapfen 7 ist eine Abgabeöffnung 18 des Grundkörpers 2 angeordnet, an welcher der Fluidkanal 17 nach aussen mündet. Die Abgabeöffnung 18 ist bezüglich der Längsachse A exzentrisch an der Stirnseite des Lagerzapfens 7 ausgebildet. Die Stirnseite des Lagerzapfens 7 weist zwei in Längsrichtung A ansteigende Rampenabschnitte 20 auf. Die Abgabeöffnung 18 ist auf einer der Rampen 20 angeordnet. Die Rampen 20 haben die Funktion, bei einer Drehung des in den Aufnahmeraum 5 eingesetzten Verschlusselements 30 einen Andruck zwischen den entsprechenden Abschnitten des Verschlusselements 30 und der Stirnseite des Lagerzapfens 7 zu erhöhen. Auf diese Weise kann eine bessere Dichtungswirkung im Bereich der Abgabeöffnung 18 erzielt werden.

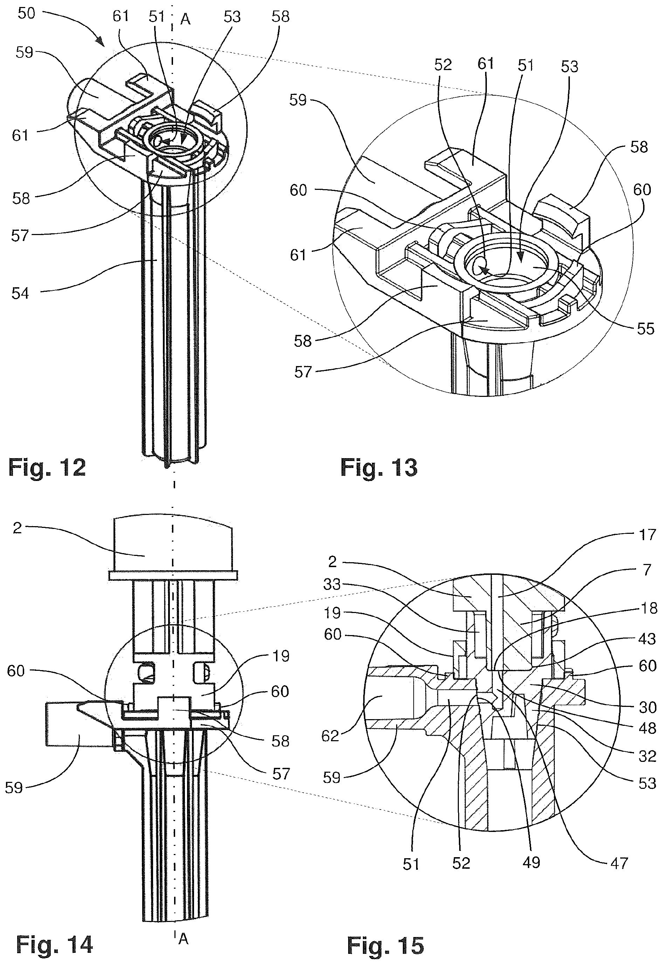

An einem vorderen Ende des Rohrabschnitts 19 sind aussenseitig zwei flanschartig auskragende, bezüglich der Längsrichtung A gegenüberliegend angeordnete, Flügel 22 ausgebildet. Die Flügel 22 bilden dabei bajonettartige Arretierungsmittel, welche mit entsprechend ausgebildeten Arretierungsmitteln einer weiteren Vorrichtung zum Eingriff gebracht werden können (siehe hierzu z.B. Fig. 14 und 15).

Im Aufnahmeraum 5 sind an einer Innenwand des Rohrabschnitts 19 umlaufend in Längsrichtung A ausgerichtete Rastkerben 23 ausgebildet. Die Rastkerben 23 sind derart geformt und angeordnet, dass bei eingesetztem Verschlusselement 30 Sperrklinken 35 des Verschlusselements 30 in die Rastkerben 23 eingreifen und somit eine relative Drehung von Grundkörper 2 und Verschlusselement 30 in eine ungewünschte Drehrichtung sperren können. Die Rastkerben 23 und die Rastklinken 35 bilden somit Teile einer Sperrvorrichtung der Abgabevorrichtung 1.

Der Rohrabschnitt 19 weist in bezüglich A radialer Richtung zwei Durchbrüche 24 auf, in welchen Anschläge 34 des Verschlusselements 30 angeordnet sind, wenn dieses in den Aufnahmeraum 5 eingesetzt ist. Die Durchbrüche 24 erstrecken sich über einen beschränkten azimutalen Winkelbereich, wodurch die relative Drehbarkeit zwischen Grundkörper 2 und Verschlusselement 30 begrenzt ist. Die Durchbrüche 24 sowie die Anschläge 34 bilden somit Teile einer Begrenzungsvorrichtung der Abgabevorrichtung 1.

Figur 6 zeigt eine Seitenansicht des Verschlusselements 30 der Abgabevorrichtung 1. Figur 7 zeigt eine weitere Seitenansicht des Verschlusselements 30 in einer Ansicht senkrecht zur Blickrichtung der Fig. 6 und Fig. 8 zeigt eine Schrägansicht des Verschlusselements 30. Figur 9 zeigt eine Draufsicht auf das Verschlusselement 30 längs der Längsachse A und Fig. 10 eine Draufsicht in entgegen gesetzter Blickrichtung. Die Fig. 6 bis 10 sind im Folgenden gemeinsam beschrieben.

Das Verschlusselement 30 weist einen weitgehend kreiszylindrischen Abschnitt 31 auf, an welchen sich in Richtung nach vorne ein kegelstumpfförmiger Abschnitt 32 anschliesst. "Vorne" und "hinten" sowie eine Anordnung der Achse A beziehen sich hier auf die Längsachse A des Grundkörpers 2, wenn das Verschlusselement 30 in betriebsbereitem Zustand am Grundkörper 2 angeordnet ist (siehe hierzu z.B. Fig. 11, 14 und 15).

Das Verschlusselement 30 ist in betriebsbereitem Zustand mit dem kreiszylindrischen Abschnitt 31 im Aufnahmeraum 5 angeordnet (siehe hierzu Fig. 11). Der kreiszylindrische Abschnitt 31 weist einen von einer Seitenwand 36 begrenzten Aufnahmeraum 33 für den Lagerzapfen 7 des Grundkörpers 2 auf. Aussenseitig an der Seitenwand 36 sind im kreiszylindrischen Abschnitt 31 die zwei Vorsprünge 34 angeordnet, welche bezüglich A gegenüberliegend und senkrecht zu A auskragen. Die Vorsprünge 34 greifen zur Begrenzung eines relativen Drehbereichs zwischen Grundkörper 2 und Verschlusselement 30 in die Durchbrüche 24 des Rohrabschnitts 19 ein, wenn das Verschlusselement 30 in betriebsbereitem Zustand im Aufnahmeraum 5 angeordnet ist (siehe hierzu Fig. 11). Zwischen den Vorsprüngen 34 ist die Seitenwand 36 des kreiszylindrischen Abschnitts 31 von hinten her unterbrochen, sodass die Vorsprünge 34 zum Einbringen in die Durchbrüche 24 federnd nach innen, d.h. zur Längsachse A hin, geschwenkt werden können.

In einem Bereich am Übergang zum kegelstumpfförmigen Abschnitt 32 sind die Sperrklinken 35 am kreiszylindrischen Abschnitt 31 angeordnet. Die Sperrklinken 35 sind dabei als federnde Zungen ausgebildet, welche sich, in Richtung von A von vorne her gesehen, weitgehend tangential im Uhrzeigersinn von der Seitenwand 36 des Abschnitts 31 erstrecken.

Der kegelstumpfförmige Abschnitt 32 weist eine nach vorne konisch zusammenlaufende Mantelfläche 37 auf und ist in Richtung nach vorne an einer Stirnseite 38 abgeschlossen. Der Abschnitt 32 ist an seiner Basis, mit welcher er an den kreiszylindrischen Abschnitt 31 angrenzt, gegenüber diesem stufenförmig abgesetzt. Auf der Mantelfläche 37 ist eine Eintrittsöffnung 49 eines Füllkanals 47 des Verschlusselements 30 ausgebildet.

Die Eintrittsöffnung 49 ist auf einer Dichtrippe 39 angeordnet, welche als konvexer Wulst den kegelstumpfförmigen Abschnitt 32 bezüglich der Längsachse A vollständig umläuft. In Längsrichtung A vor und hinter der Dichtrippe 39 können weitere umlaufende Rippen z.B. zur Verrastung oder zur zusätzlichen Dichtung vorgesehen sein. Die Dichtrippe 39 stellt sicher, dass im Bereich um die Eintrittsöffnung 49 eine ausreichende Dichtigkeit sichergestellt werden kann, wenn der kegelstumpfförmige Abschnitt 32 in einem Sitz z.B. einer Transfervorrichtung für ein Fluid eingesetzt ist. Vor der Dichtrippe 39 sind, auf bezüglich der Längsachse A gegenüberliegenden Seiten, in Längsrichtung ausgerichtete Nuten 44 auf der Mantelfläche 37 ausgebildet. Ein hinteres Ende der Nuten 44 ist dabei von der Dichtrippe 39 beabstandet. An der Stirnseite 38 ist eine Austrittsöffnung 41 des Abgabekanals 40 ausgebildet. Die Austrittsöffnung 41 ist exzentrisch bezüglich A angeordnet.

Im Aufnahmeraum 33 ist ein Sitz 43 für den Lagerzapfen 7 als Vertiefung in einem Boden des Aufnahmeraums 33 ausgebildet. Der Lagerzapfen 7 ist mit seiner Stirnseite im Sitz 43 angeordnet, wenn sich das Verschlusselement 30 in betriebsbereitem Zustand im Aufnahmeraum 5 befindet (siehe hierzu z.B. Fig. 15 und 18). Am Boden des Sitzes 43 ist eine Eintrittsöffnung 42 des Abgabekanals 40 sowie eine Austrittsöffnung 48 des Füllkanals 47 ausgebildet. Die beiden Öffnungen 42 und 48 sind exzentrisch bezüglich A, im gleichen radialen Abstand von A und unter einem azimutalen Winkel von 90 Grad zueinander angeordnet. Insbesondere ist die Öffnung 42 bezüglich A fluchtend mit der Öffnung 41 angeordnet. Am Boden des Sitzes 43 sind zwei in Längsrichtung A ansteigende Rampenabschnitte 46 angeordnet, welche mit den Rampenabschnitten 20 des Lagerzapfens 7 dichtend zusammenwirken.

Figur 11 zeigt eine Seitenansicht des Abgabebereichs 4 der Abgabevorrichtung 1 mit dem im Aufnahmeraum 5 angeordnete Verschlusselement 30. Das Verschlusselement 30 ist mit dem Abschnitt 31 im Aufnahmeraum 5 angeordnet. Die Vorsprünge 34 im kreiszylindrischen Abschnitt 31 greifen zur Begrenzung eines relativen Drehbereichs zwischen Grundkörper 2 und Verschlusselement 30 in die Durchbrüche 24 des Rohrabschnitts 19 ein. Der kegelstumpfförmige Abschnitt 32 ragt dabei in Richtung von A nach vorne aus dem Aufnahmeraum 5 und über den Rohrabschnitt 19 hinaus. Der kegelstumpfförmige Abschnitt 32 bildet ein Kopplungsmittel der Abgabevorrichtung 1 zum Ankoppeln an eine weitere Vorrichtung, welche einen entsprechenden Sitz aufweist (siehe hierzu z.B. Fig. 12-15).

Das Verschlusselement 30 ist mit einer Kanüle 45 versehen. Die Kanüle 45 ist durch die Austrittsöffnung 41 direkt in den Abgabekanal 40 eingesetzt. Die Kanüle 45 wird dabei bevorzugt direkt bei der Herstellung des Verschlusselements 30 umspritzt. In diesem Fall wird der Abgabekanal 40 von der Kanüle 45 im Verschlusselement 30 ausgebildet. Die Kanüle 45 erstreckt sich parallel zur Längsrichtung A von der Stirnseite 38 nach vorne (siehe hierzu auch Fig. 16 und 19).

Figur 12 zeigt eine Schutzkappe 50 für die Abgabevorrichtung 1 mit einem Transferkanal 51 zur Zuführung eines Fluids. Figur 13 zeigt eine Ausschnittsansicht eines Sitzes 53 der Schutzkappe 50 für ein Kopplungsmittel der Abgabevorrichtung 1. Figuren 12 und 13 sind im Folgenden gemeinsam beschrieben.

Der Sitz 53 erlaubt das Einstecken der Abgabevorrichtung 1 in die Schutzkappe 50. Der Sitz 53 ist an einer Einstecköffnung einer länglichen Hülse 54 zur Aufnahme der Kanüle 45 ausgebildet. Der Sitz 53 ist derart ausgebildet, dass die Abgabevorrichtung 1 mit dem kegelstumpfförmigen Abschnitt 32 des Verschlusselements 30 in die Schutzkappe 50 eingesteckt werden kann (siehe z.B. Fig. 15 und 16). Der kegelstumpfförmige Abschnitt 32 bildet somit ein Kopplungsmittel der Abgabevorrichtung 1. Die Hülse 54 ist zur Aufnahme der am Verschlusselement 30 angebrachten Kanüle 45 ausgebildet. Die Hülse 54 ist aussenseitig mit Längsrippen versehen, welche eine Aussenwand versteifen. In eingestecktem Zustand ist die Hülse 54 weitgehend koaxial mit der Längsachse A angeordnet. "Vorne" und "hinten" sowie eine Anordnung der Achse A beziehen sich hier auf die Längsachse A des Grundkörpers 2, wenn die Abgabevorrichtung 1 auf vorgesehene Weise in die Schutzkappe 50 eingesteckt ist.

Der Sitz 53 weist eine zum kegelstumpfförmigen Abschnitt 32 komplementäre konische Form auf. Eine Dichtkerbe 55 ist derart an einer Innenfläche des Sitzes 53 bezüglich A vollständig umlaufend angeordnet, dass bei vollständigem Einbringen des kegelstumpfförmigen Abschnitts 32 dessen Dichtrippe 39 dichtend in der Dichtkerbe 55 sitzt. Sofern weitere Rippen zur Verrastung oder zusätzlichen Abdichtung vorhanden sind, kann der Sitz 53 entsprechende Kerben aufweisen. Ebenso weist der Sitz 53 Längsrippen 56 in Richtung von A auf, welche als Verdrehsicherung in die Nuten 44 des kegelstumpfförmigen Abschnitts 32 eingreifen können (siehe hierzu z.B. Fig. 16 und 19). Der kegelstumpfförmige Abschnitt 32 kann somit in Richtung von A in den Sitz 53 eingesteckt werden, wobei die Dichtrippe 39 in der Dichtkerbe 55 verrastet.

Aussenseitig an der Schutzkappe 50, in einem Längenbereich des Sitzes 53, ist ein umlaufender, plattenförmiger Flansch 57 ausgebildet. Am Flansch 57 sind zwei Arretierungsmittel 58 angeordnet, welche komplementär zu den als Flügel 22 ausgebildeten Arretierungsmitteln des Grundkörpers 2 sind. Die Arretierungsmittel 58 sind dabei als Hinterschnitte bezüglich A derart ausgebildet, dass die Flügel 22 durch Drehung des Grundkörpers 2 um A zum Eingriff mit den Arretierungsmitteln 58 gebracht werden können (d.h. die Hinterschnitte sind in Drehrichtung seitlich offen). Flügel 22 und Hinterschnitte 58 bilden somit eine bajonettartige Arretierung gegen ein Abziehen des kegelstumpfförmigen Abschnitts 32 aus dem Sitz 53.

In der Dichtkerbe 55 ist eine Füllöffnung 52 des Transferkanals 51 ausgebildet. Die Füllöffnung 52 ist derart angeordnet, dass die Eintrittsöffnung 49 des Füllkanals 47 des Verschlusselements 30 dichtend an die Füllöffnung 52 anschliesst, wenn der kegelstumpfförmige Abschnitt 32 im Sitz 53 angeordnet ist. Der Transferkanal 51 setzt sich in der Schutzkappe 50 in radialer Richtung senkrecht zu A in einem Anschlussstutzen 59 fort. Der Anschlussstutzen 59 dient zum Anschluss der Schutzkappe 50 an eine Fluidquelle oder eine Transfervorrichtung zum Transfer eines Fluids z.B. aus einer Glasfiole. Eine derartige Transfervorrichtung bzw. ein derartiges Transfersystem mit mehreren Transfervorrichtungen ist in der WO 2013/024120 A1 beschrieben. Der Anschlussstutzen 59 weist hierzu einen Aufnahmeraum 62 für ein entsprechendes Kopplungselement der Fluidquelle oder der Transfervorrichtung auf (siehe z.B. Fig. 15 und 18).

Der Anschlussstutzen 59 ist im Wesentlichen in der Ebene des Flansches 57 angeordnet und erstreckt sich in radialer Richtung senkrecht zu A. Zusätzlich können am Flansch 57 in Richtung des Anschlussstutzens 59 Haltemittel 61 ausgebildet sein, mit welchen die Schutzkappe 50 z.B. an einer angeschlossenen Transfervorrichtung fixiert bzw. abgestützt oder anderweitig gehaltert werden kann.

Zwischen den Arretierungsmitteln 58 sind Rampen 60 am Flansch 57 ausgebildet, welche sich in azimutaler Richtung um A erstrecken. Die Rampen 60 sind bezüglich A teilkreisförmig ausgebildet und haben eine in Richtung von A zunehmende Höhe. Die Höhenzunahme beider Rampen 60 erfolgt mit demselben Drehsinn bezüglich A. Die Rampen 60 sind derart angeordnet, dass durch Drehen des Grundkörpers 2 um A die Flügel 22 auf die Rampen 60 aufgleiten können. Auf diese Weise kann aus der relativen Drehung um A zwischen Schutzkappe 50 und Abgabevorrichtung 1 eine Kraft in Richtung von A erzeugt werden. Diese Kraft dient dazu, den kegelstumpfförmigen Abschnitt 32 vom Sitz 53 abzuheben, sodass die Abgabevorrichtung 1 problemlos aus der Schutzkappe 50 ausgebracht werden kann. Insbesondere kann auf diese Weise eine Verrastung der Dichtrippe 39 in der Dichtkerbe 55 auf einfache Art durch die ohnehin erforderliche Drehung des Grundkörpers 2 zum Lösen des Eingriffs der Flügel 22 in die Arretierungsmittel 58 gelöst werden (siehe hierzu auch Fig. 16 und 19).

Figur 14 zeigt eine Ausschnittsansicht des mit der Schutzkappe 50 versehenen Abgabebereichs 4 der Abgabevorrichtung 1 mit dem Verschlusselement 30 in einer Füllstellung. Figur 15 zeigt eine Teilansicht eines Querschnitts des Abgabebereichs 4 der Fig. 14 in einer Schnittebene welche A umfasst. Figur 16 zeigt eine Querschnittsansicht der Ansicht der Fig. 14 in einer Ebene welche A umfasst und senkrecht zur Schnittebene der Fig. 15 angeordnet ist. Figuren 14 bis 16 sind im Folgenden gemeinsam beschrieben.

Die Flügel 22 sind mit den Arretierungsmitteln 57 der Schutzkappe 50 im Eingriff und arretieren die Abgabevorrichtung 1 gegen ein Abziehen aus der Schutzkappe 50 in Richtung von A. Der kegelstumpfförmige Abschnitt 32 des Verschlusselements 30 ist im Sitz 53 angeordnet.

Wie aus Fig. 15 ersichtlich ist, schliesst die Eintrittsöffnung 49 des Füllkanals 47 an die Füllöffnung 52 des Transferkanals 51 an. Gleichzeitig schliesst die Austrittsöffnung 48 des Füllkanals 47 an die Abgabeöffnung 18 des Grundkörpers 2 an. Es besteht somit eine Fluidverbindung zwischen dem Transferkanal 51 und dem Aufnahmeraum 14 des Grundkörpers 2 der Abgabevorrichtung 1. Diese Stellung des Verschlusselements 30 ist vorliegend als Füllstellung bezeichnet, da ein über den Anschlussstutzen 59 zugeführtes Fluid in den Aufnahmeraum 14 gefüllt werden kann bzw. von der Abgabevorrichtung 1 aufgezogen werden kann. Im Anschlussstutzen 59 ist im Querschnitt der Aufnahmeraum 62 für ein Kopplungsmittel z.B. eine Fluidquelle ersichtlich.

Weiter ist in Fig. 15 ersichtlich, dass der kreiszylindrische Abschnitt 31 im Aufnahmeraum 5 des Rohrabschnitts 19 angeordnet ist und der Lagerzapfen 7 in den Sitz 43 am Boden des Aufnahmeraums 33 des Verschlusselements 30 ragt.

In Fig. 16 ist die exzentrisch parallel zu A sich erstreckende Kanüle 45 im Innenraum der Hülse 54 ersichtlich. Die Kanüle 45 erstreckt sich im Abgabekanal 40 im Wesentlichen durch das gesamte Verschlusselement 30. Wie sich aus dem Bereich am Lagerzapfen 7 erschliesst, ist in der Füllstellung eine Fluidverbindung zwischen dem Fluidkanal 17 des Grundkörpers 2 und dem Abgabekanal 40 des Verschlusselements 30 unterbrochen. Die Füllstellung entspricht somit einer Verschlussstellung.

Figur 17 zeigt dieselbe Ausschnittsansicht wie Fig. 14, wobei das Verschlusselement 30 in einer Abgabestellung ist, kurz vor dem vollständigen Ausbringen aus der Schutzkappe 50. Figur 18 zeigt eine Teilansicht eines Querschnitts des Abgabebereichs 4 der Fig. 17 in einer Schnittebene, welche A umfasst. Figur 19 zeigt eine Querschnittsansicht der Ansicht der Fig. 17 in einer Ebene welche A umfasst und senkrecht zur Schnittebene der Fig. 18 angeordnet ist. Figuren 17 bis 19 sind im Folgenden gemeinsam beschrieben.

Im Unterschied zu den Fig. 14 bis 16 ist der Grundkörper 2 bezüglich der Schutzkappe 50 um etwa 90 Grad um die Längsachse A gedreht. Da das Verschlusselement 30 verdrehsicher (durch den Eingriff der Rippen 56 in die Nuten 44 gesichert) im Sitz 53 angeordnet ist, ist somit auch das Verschlusselement 30 bezüglich dem Grundkörper 2 um A gedreht.

Zum einen gelangt aufgrund der relativen Drehung um A die Abgabeöffnung 18 des Grundkörpers 2 auf die Eintrittsöffnung 42 des Abgabekanals 40 des Verschlusselements 30. In dieser Stellung ist somit eine durchgehend Fluidverbindung zwischen dem Aufnahmeraum 14 und der Kanüle 45 hergestellt. Diese Stellung der Verschlusselements 30 ist vorliegend als Abgabestellung bezeichnet.

Zum anderen sind aufgrund der relativen Drehung die Flügel 22 aus den Arretierungsmitteln 58 ausgebracht worden und dabei auf die Rampen 60 aufgeglitten. Durch das Aufgleiten der Flügel 22 auf die Rampen 60 wird das Verschlusselement 30 über den Grundkörper 2 aus dem Sitz 53 gehoben (in Richtung des Pfeils). Die Nuten 44 und die Rippen 56 sind dabei derart ausgebildet, dass während dieser Abhebebewegung die Verdrehsicherung zwischen Verschlusselement 30 und Sitz 53 bestehen bleibt.

Die relative Drehung zwischen Grundkörper 2 und Verschlusselement 30 ist aufgrund der in den Durchbrüchen 24 angeordneten Vorsprünge 34 begrenzt. Aus Fig. 18 geht zudem hervor, dass in der Abgabestellung eine Fluidverbindung zwischen dem Füllkanal 47 und dem Fluidkanal 17 bzw. dem Aufnahmeraum 14 unterbrochen ist.

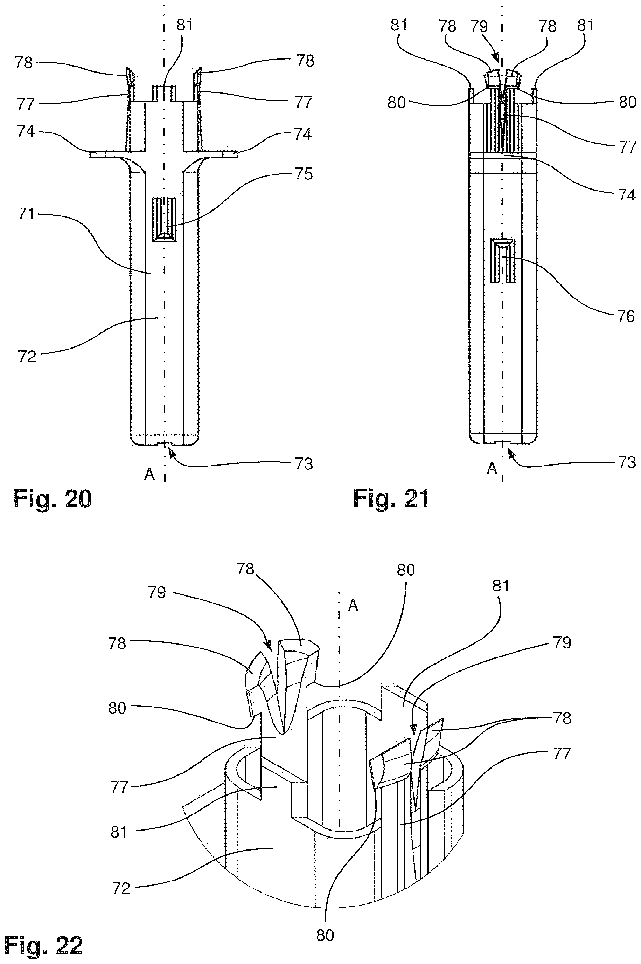

Figur 20 zeigt eine Seitenansicht eines Schutzkörpers 71 einer Schutzvorrichtung 70 (siehe z.B. Fig. 23) einer erfindungsgemässen Abgabevorrichtung 1. Figur 21 zeigt eine weitere Seitenansicht des Schutzkörpers 71 der Fig. 20 und Fig. 22 zeigt eine Ausschnittsansicht von den als Rastzungen 77 ausgebildeten Auslösemitteln des Schutzkörpers 71 der Fig. 20. Die Fig. 20-22 sind im Folgenden gemeinsam beschrieben.

Der Schutzkörper 71 umfasst eine rohrförmige Hülse 72 mit einem an den Kanten abgerundeten, weitgehend quadratischen Querschnitt. Es versteht sich, dass diese Ausbildung des Querschnitts nur eine exemplarische Lösung aus einer Vielzahl von möglichen alternativen Ausbildungen darstellt. Insbesondere kann der Querschnitt z.B. auch kreisförmig und bevorzugt an den kreiszylindrischen Abschnitt 3 des Grundkörpers 2 angepasst sein. Der Schutzkörper 71 weist an einem in Längsrichtung A vorderen Ende eine Öffnung 73 auf (siehe z.B. Fig. 24) durch welche der Rohrabschnitt 19 des Grundkörpers 2 der Abgabevorrichtung 1 hindurch treten kann, wenn der Schutzkörper 71 in einer Bereitschaftsstellung am Grundkörper 2 angeordnet ist. Die Längsachse A bezieht sich auf die Lage der Längsachse A des Grundkörpers 2, wenn der Schutzkörper 71 am Grundkörper 2 angeordnet ist.

In einem hinteren Bereich sind aussenseitig bezüglich A senkrecht gegenüberliegend auskragende Flügel 74 als Fingerflansch ausgebildet. Die Flügel 74 sind dabei derart angeordnet, dass sie entsprechend den Flügeln 8 des Grundkörpers 2 ausgerichtet sind, wenn der Schutzkörper 71 am Grundkörper 2 angeordnet ist.

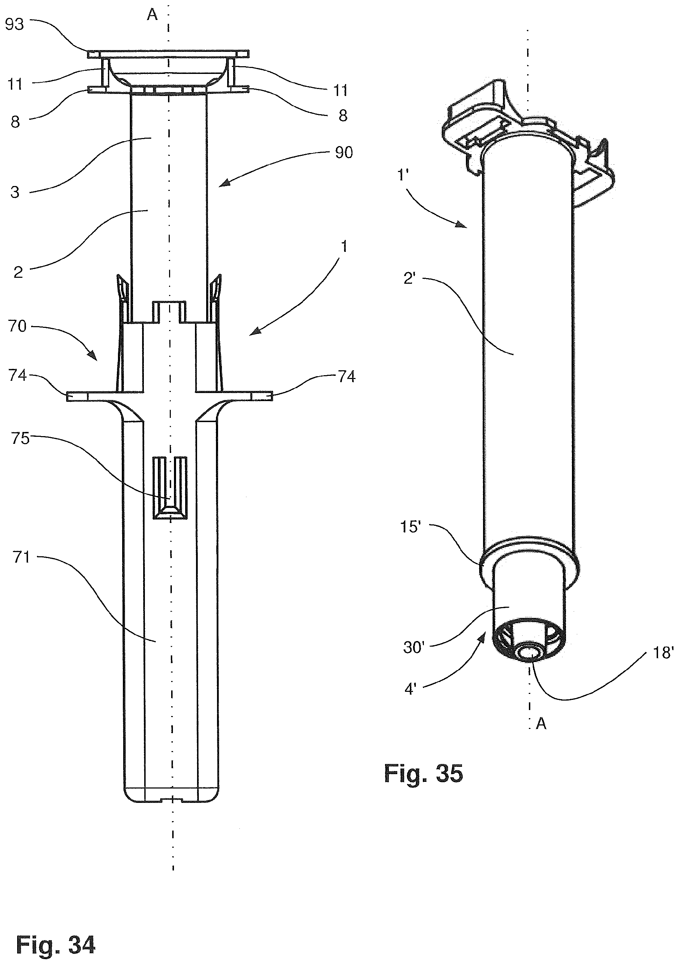

An den Seitenwänden der Hülse 72 sind jeweils Paare von Rastzungen 75 und 76 angeordnet. Die beiden Rastzungen jeweils eines der Paare sind auf bezüglich A gegenüberliegenden Seitenwänden der Hülse 72 angeordnet, wobei eine an einem freien Ende der Rastzungen angeordnete Rastkante nach innen ragt. Das andere Paar ist an den weiteren beiden gegenüberliegenden Seitenwänden angeordnet. Das Paar 75 ragt mit einem freien Ende nach vorne, während das Paar 76 in Längsrichtung A weiter vorne am Schutzkörper 71 angeordnet ist und mit den freien Enden nach hinten ragt. Die freien Enden der Rastzungen 75 und 76 sind dabei derart zueinander am Schutzkörper 71 angeordnet, dass in einer Schutzstellung des Schutzkörpers 71, d.h. wenn dieser bezüglich des Grundkörpers 2 nach vorne verschoben ist (siehe hierzu Fig. 34), die Rastzungen 75 und 76 an einem auskragenden Rand der Abschlussplatte 15 bezüglich der Längsrichtung A beidseitig verrasten können. Die Rastzungen 75 sperren den Schutzkörper 71 dabei in Richtung nach vorne, während ihn die Rastzungen 76 in Richtung nach hinten sperren. Der Schutzkörper 71 ist in der Schutzstellung somit fest am Grundkörper 2 arretiert. Auf diese Weise ist sichergestellt, dass die Abgabevorrichtung nach einem Übergang der Schutzvorrichtung 70 in die Schutzstellung nicht erneut benutzt werden kann.

Ein Längenbereich der Hülse 72 vor den Rastzungen 76 ist dabei derart bemessen, dass die Kanüle 45 in der Schutzstellung vollständig im Inneren der Hülse 72 angeordnet ist.

In einem Endabschnitt der Hülse 72 hinter dem Flügeln 74 ist an den Seitenwänden, an welchen die Flügel 74 angeordnet sind, jeweils eine nach hinten ragende Rastzunge 77 ausgebildet. Die Rastzungen 77 sind baugleich ausgeführt, weshalb im Folgenden nur eine der Rastzungen 77 exemplarisch beschrieben ist.

Die Rastzunge 77 ragt mit ihrem freien Längsende in Richtung von A über die Hülse 72 hinaus und kann bezüglich der Hülse 72 mit dem freien Ende zur Längsachse A hin und von dieser weg verschwenkt werden. Das freie Längsende der Rastzunge 77 weist jeweils zwei Flügel 78 auf, welche durch einen zur Hülse 72 hin, d.h. nach vorne, zulaufenden Spalt 79 voneinander getrennt sind. Aufgrund des Spalts 79 können die Flügel 78 in Richtung senkrecht zu A relativ zueinander, insbesondere zueinander hin, bewegt werden. Die Flügel 78 können dabei federelastisch mit der übrigen Rastzunge 77 verbunden sein, sodass nach einer Auslenkung eine Rückstellung in eine Ausgangsstellung erfolgt.

Die Flügel 78 weisen jeweils an einer der Hülse 72 zugewandten Seite eine Abstufung 80 auf, welche als Rastkante der Rastzunge 77 zum Verrasten an den Rastkanten 10 des Grundkörpers 2 dient. Die Rastzungen 77 bilden ein Auslösemittel der Schutzvorrichtung 70.

In einer Projektion in Richtung von A ragen die Flügel 78 bezüglich A nach aussen über die übrige Rastzunge 77 hinaus. Dabei weisen die Flügel 78 eine Schalenform auf, welche bezüglich A nach aussen gewölbt ist. Die Flügel 78 bilden somit eine endseitige Aufweitung der Rastzungen 77. Aufgrund des Spalts 94 ist diese Aufweitung deformierbar. An den weiteren beiden Seitenwänden erstrecken sich Wandabschnitte 81 nach hinten über die Hülse 72 hinaus, welche zumindest in der Bereitschaftsstellung in entsprechende Führungsnuten am Grundkörper 2 eingreifen können.

Figur 23 zeigt eine Aussenansicht einer Abgabevorrichtung 1, welche einen mit der Schutzvorrichtung 70 versehenen Grundkörper 2 aufweist. Figur 24 zeigt eine Querschnittsansicht der Ansicht der Fig. 23 in einer Ebene, längs welcher die Flügel 8 bzw. 74 ausgerichtet sind und in welcher die Längsachse A angeordnet ist. Figur 25 zeigt eine Teilansicht des Querschnitts der Fig. 24 an einem hinteren Ende der Abgabevorrichtung 1. Figur 26 zeigt eine Querschnittsansicht in einer Schnittebene im Bereich der Rastzunge 77, wie sie in Fig. 25 eingezeichnet ist. Figuren 23 bis 26 sind im Folgenden gemeinsam beschrieben.

Eine Betätigungsvorrichtung 90 der Abgabevorrichtung 1 umfasst einen Kolben 91, eine starr mit dem Kolben 91 verbundene Kolbenstange 92 sowie eine schalenförmige Fingerplatte 93. Die schalenförmige Fingerplatte 93 weist eine zum Grundkörper 2 hin konvex gewölbte Aussenfläche 95 auf. Der Kolben 91 und die Fingerplatte 93 sind an gegenüberliegenden Längsenden der Kolbenstange 92 angeordnet. Die Betätigungsvorrichtung 90 ist mit dem Kolben 91 in den Aufnahmeraum 14 des Grundkörpers 2 vollständig eingeschoben, wobei eine Längsachse der Kolbenstange 92 koaxial mit der Längsachse A angeordnet ist. Die Betätigungsvorrichtung 90 ist derart bemessen, dass die Fingerplatte 93 in dieser Stellung an den Stützstrukturen 11 des Grundkörpers 2 anliegt.

Der Schutzkörper 71 ist von vorne her über den Grundkörper 2 geschoben, sodass der Rohrabschnitt 19 mit darin angeordnetem Verschlusselement 30 und daran angeordneter Kanüle 45 durch die Öffnung 73 in Längsrichtung A noch vorne hinaus ragt. An der Anschlussplatte 15 abgestützt ist eine Schraubenfeder 82 zwischen dem Grundkörper 2 und dem Schutzkörper 71 angeordnet. Der Rohrabschnitt 19 dient dabei als Sitz für die Schraubenfeder 82.

Die Rastzungen 77 sind durch die Durchbrüche 9 der Flügel 8 des Grundkörpers 2 geführt und mit ihren Rastkanten 80 an den Rastkanten 10 des Grundkörpers 2 abgestützt, d.h. verrastet. Die Flügel 78 ragen dabei durch Durchbrüche 94 der Fingerplatte 93, welche sich in Richtung von A durch die Fingerplatte 93 erstrecken. Die Durchbrüche 94 sind dabei derart bemessen, dass die Flügel 78 in dieser Stellung an einem bezüglich A äusseren Rand der Durchbrüche 94 anliegen. Die Verrastung der Rastzungen 77 an den Rastkanten 10 des Grundkörpers 2 kann aufgrund der Abstützung der Flügel 78 am Rand der Durchbrüche 94 nicht gelöst werden. Die Fingerplatte 93 mit den daran ausgebildeten Durchbrüchen 94 bildet somit ein Sicherungsmittel der Abgabevorrichtung 1, mit welchem die Schutzvorrichtung 70 in einen gesicherten Zustand gebracht werden kann. Ein in Richtung von A hinterster Bereich der Flügel 78 erstreckt sich dabei in einer Projektion längs A über einen lichten Querschnitt der Durchbrüche 94 hinaus.

Der Schutzkörper 71 befindet sich somit in einer Bereitschaftsstellung, wobei die Schutzvorrichtung 70 in einem gesicherten Zustand ist.

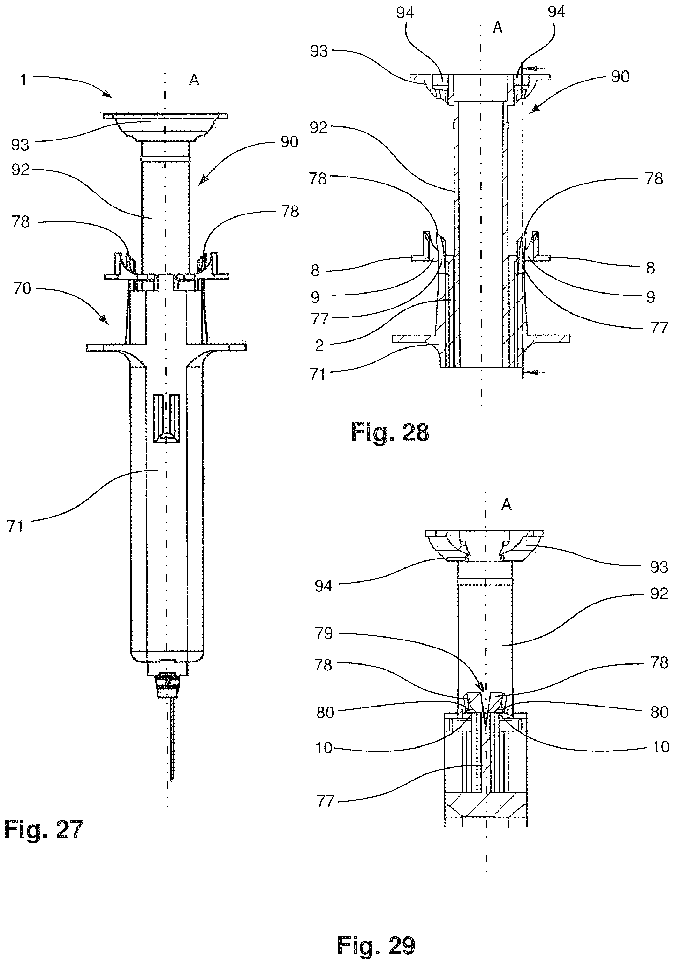

Figur 27 zeigt eine Aussenansicht der Abgabevorrichtung 1, wobei die Schutzvorrichtung 70 in einem ungesicherten Zustand ist. Die Abgabevorrichtung 1 ist dabei teilweise aufgezogen. Figur 28 zeigt eine Teilansicht eines Querschnitts an einem hinteren Ende der Abgabevorrichtung 1. Figur 29 zeigt eine Querschnittsansicht in einer Schnittebene im Bereich der Rastzunge 77, wie sie in Fig. 28 eingezeichnet ist. Figuren 27 bis 29 sind im Folgenden gemeinsam beschrieben.

Die Fingerplatte 93 ist zusammen mit der Kolbenstange 92 und dem Kolben 91 in Richtung von A nach hinten aus dem Grundkörper 2 verschoben. Die Flügel 78 der Rastzungen 77 sind aus den Durchbrüchen 94 der Fingerplatte 94 ausgebracht. Die Flügel 78 einer Rastzunge 77 wurden dabei zueinander hin gezwungen, um durch den entsprechenden Durchbruch 94 hindurch gleiten zu können. Der Spalt 79 erlaubt dabei die erforderliche Deformation der durch die Flügel 78 gebildeten Aufweitung der Rastzunge 77. Nach dem Ausbringen sind die Flügel 78 wieder in die ursprüngliche Stellung zurück gestellt.

Die Rastzungen 77 sind mit den Rastkanten 80 an den Rastkanten 10 des Grundkörpers 2 abgestützt. Da die Flügel 78 nicht mehr an den Durchbrüchen 94 abgestützt sind, können die Rastzungen 77 nach aussen, d.h. von der Längsachse A in radialer Richtung weg, bewegt bzw. gebogen werden. Mit dem von der Fingerplatte 93 mit den daran ausgebildeten Durchbrüchen 94 gebildeten Sicherungsmittel kann die Schutzvorrichtung 70 somit in einen ungesicherten Zustand gebracht werden.

Der Schutzkörper 71 befindet sich somit weiterhin in der Bereitschaftsstellung, wobei die Schutzvorrichtung 70 aber in einem ungesicherten, zur Auslösung bereiten Zustand ist.

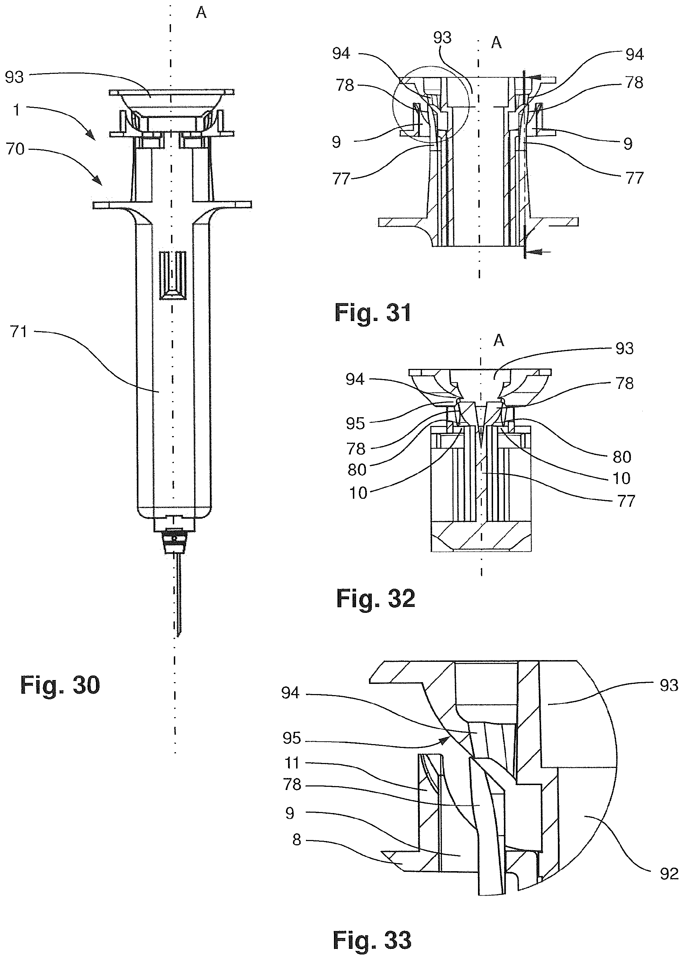

Figur 30 zeigt eine Aussenansicht der Abgabevorrichtung 1 im Moment der Auslösung der Schutzvorrichtung 70. Figur 31 zeigt eine Teilansicht eines Querschnitts an einem hinteren Ende der Abgabevorrichtung 1. Figur 32 zeigt eine Querschnittsansicht in einer Schnittebene im Bereich der Rastzunge 77 wie sie in Fig. 31 eingezeichnet ist. Figur 33 zeigt eine vergrösserte Ausschnittsansicht der Darstellung der Fig. 31 im Bereich der Flügel 78 einer der Rastzungen 77. Figuren 30 bis 33 sind im Folgenden gemeinsam beschrieben.

Das Verschlusselement 30 befindet sich in einer Abgabestellung, wobei der Kolben 91 im Wesentlichen vollständig in den Aufnahmeraum 14 des Grundkörpers 2 eingeschoben ist. Die schalenförmige Fingerplatte 93 ist längs A zum Grundkörper 2 hin verschoben, sodass eine Aussenseite der Fingerplatte 93 von hinten her an die Flügel 78 der Rastzungen 77 stösst.

Da sich der in Richtung von A hinterste Bereich der Flügel 78 in einer Projektion längs A über einen lichten Querschnitt der Durchbrüche 94 hinaus erstreckt, gleiten die Flügel 78 auf die Aussenseite 95 der Fingerplatte 93 auf. Wird die Fingerplatte 93 weiter zum Grundkörper 2 hin gedrückt, zwingt die konvex geformte Aussenfläche 95 der Fingerplatte 93 die Flügel 78 in radialer Richtung von A weg nach aussen. Die Aussenfläche 95 bildet somit eine Steuerfläche zum Auslösen der Rastzungen 77. Die Durchbrüche 9 in den Flügel 8 des Grundkörpers 2 sind hierzu entsprechend bemessen, sodass die Rastzungen 77 genügend weit nach aussen bewegt werden können. Auf diese Weise wird eine Verrastung der Rastkanten 80 an den Rastkanten 10 gelöst und die Schutzvorrichtung 70 ausgelöst.

Der Schutzkörper 71 ist nach der Auslösung von den Rastzungen 77 nicht mehr am Grundkörper 2 arretiert und ist grundsätzlich frei, um von der Federkraft der Feder 82 gegenüber dem Grundkörper 2 nach vorne in die Schutzstellung verschoben zu werden. Es versteht sich, dass der Schutzkörper 71 von einem Benutzer am Fingerflansch 74 in der Bereitschaftsstellung gehalten werden kann, auch wenn die Schutzvorrichtung 70 bereits ausgelöst ist.

Figur 34 zeigt eine Aussenansicht der Abgabevorrichtung 1, wobei die Schutzvorrichtung 70 in der Schutzstellung ist.

Der Schutzkörper 71 ist relativ zum Grundkörper 2 längs A ganz nach vorne verschoben, sodass der rohrförmige Abschnitt 3 des Grundkörpers 2 weitgehend frei liegt. Der Schutzkörper 71 ist über die Rastzungenpaare 75 und 76 an der Abschlussplatte 15 verrastet. Die Kanüle 45 ist vollständig innerhalb des Schutzkörpers 71 angeordnet, sodass keine Verletzungsgefahr für einen Benutzer besteht. Die Fingerplatte 93 ist vollständig auf die Stützstruktur 11 abgesenkt, wobei der Kolben 91 im Aufnahmeraum 14 ganz noch vorne verschoben ist. Die Abgabevorrichtung 1 ist somit nach einer Benutzung entleert. Indem der Schutzkörper 71 am Grundkörper 2 verrastet ist, kann die Abgabevorrichtung 1 nicht mehr wieder verwendet werden und muss entsorgt werden.

Figur 35 zeigt eine weitere Ausführungsform eines Grundkörpers 2' einer unvollständig dargestellten Abgabevorrichtung 1' (ohne Betätigungsvorrichtung und Schutzvorrichtung), welcher zur Anwendung mit einer Schutzvorrichtung 70 geeignet ist.

Der Grundkörper 2' ist zumindest von hinten her bis zu einer Abschlussplatte 15' identisch wie der Grundkörper 2 der Abgabevorrichtung 1 ausgebildet. Anstelle des Verschlusselements 30 ist im Abgabebereich 4' eine herkömmliche Luer-Lock Kupplung 30' ausgebildet. Eine Abgabeöffnung 18', welche an einer Stirnseite eines Konus der Kupplung 30' ausgebildet ist, kommuniziert über einen innen liegenden Fluidkanal mit einem Aufnahmeraum im Grundkörper 2'. Der Grundkörper 2' kann analog der Abgabevorrichtung 1 mit einer Schutzvorrichtung 70 versehen werden. Dabei ist die Öffnung 73 des Schutzkörpers 71 in diesem Fall derart bemessen, dass die Luer-Kupplung 30' zumindest teilweise durch sie hindurchragen kann. Die Luer-Kupplung 30' kann als Sitz für die Schraubenfeder 82 dienen. Aus der vorhergehenden Beschreibung erschliesst sich unmittelbar, wie die Schutzvorrichtung 70 am Grundkörper 2' anzuordnen ist.

## Patentansprüche

1. Abgabevorrichtung (1, 1') zur Abgabe eines Fluids, insbesondere zur medizinischen Anwendung, vorzugsweise eine Injektionsspritze, umfassend:
- einen Grundkörper (2, 2') mit einer Längsachse (A),
- einen im Grundkörper (2, 2') ausgebildeten Aufnahmeraum (14) für das Fluid,
- einen Abgabebereich (4, 4'), welcher zur Anordnung einer mit dem Aufnahmeraum (14) kommunizierende Kanüle (45) ausgebildet ist oder in welchem eine mit dem Aufnahmeraum (14) kommunizierende Kanüle (45) angeordnet ist, durch welche das Fluid aus dem Aufnahmeraum (14) abgebbar ist,
wobei die Abgabevorrichtung (1) eine Schutzvorrichtung (70) mit einem am Grundkörper (2) angeordneten starren Schutzkörper (71) umfasst, wobei der Schutzkörper (71) derart ausgebildet und bezüglich dem Grundkörper (2) derart zwischen einer Bereitschaftsstellung und einer Schutzstellung beweglich ist, dass die im Abgabebereich (4, 4') angeordnete oder anordenbare Kanüle (45) in der Bereitschaftsstellung vollständig freigelegt ist und sich der Schutzkörper (71) in der Schutzstellung vom Grundkörper (2) vollständig über die im Abgabebereich (4, 4') angeordnete oder anordenbare Kanüle (45) hinaus erstreckt, wobei ein von einem Benutzer direkt oder indirekt auslösbares Auslösemittel (77) vorhanden ist, mit welchem ein Übergang des Schutzkörpers (71) aus der Bereitschaftsstellung in die Schutzstellung auslösbar ist,
wobei das Auslösemittel (77) wenigstens eine Rastzunge (77) umfasst, welche am Schutzkörper (71) ausgebildet ist und in der Bereitschaftsstellung des Schutzkörpers (71) an einer Rastkante (10) des Grundkörpers (2) verrastet ist,
wobei ein Sicherungsmittel vorhanden ist, mit welchem die Schutzvorrichtung (70) in einen gesicherten und einen ungesicherten Zustand bringbar ist, wobei die Schutzvorrichtung (70) im gesicherten Zustand gegen eine Auslösung des Übergangs von der Bereitschaftsstellung in die Schutzstellung gesichert ist, und im ungesicherten Zustand der Übergang auslösbar ist,
wobei das Sicherungsmittel derart ausgebildet ist, dass im gesicherten Zustand der Schutzvorrichtung (70) das Auslösemittel (77) vom Sicherungsmittel gegen eine Auslösung gesichert ist,
wobei die Abgabevorrichtung (1, 1') eine Betätigungsvorrichtung (90) mit einem im Aufnahmeraum beweglichen Kolben (91) aufweist, welcher zum Einbringen des Fluids in den Aufnahmeraum (14) aus einer eingeschobenen Stellung in eine ausgezogene Stellung und zum Ausbringen in umgekehrter Richtung beweglich ist,
**dadurch gekennzeichnet, dass**
die Betätigungsvorrichtung (90) derart als Sicherungsmittel ausgebildet ist, das die Schutzvorrichtung (70) im eingeschobenen Zustand des Kolbens (91) in den gesicherten Zustand bringbar ist,
wobei die Betätigungsvorrichtung (90) eine, insbesondere starr mit dem Kolben (91) verbundene, Fingerplatte (93) zum Bewegen des Kolbens (91) aufweist, welche als Sicherungsmittel ausgebildet ist,
wobei das Sicherungsmittel als wenigstens ein Durchbruch (94) in der Fingerplatte (93) ausgebildet ist, durch welchen die wenigstens eine Rastzunge (77), hindurchragen kann, wenn der Kolben (91) in der eingeschobenen Stellung ist, wobei der Durchbruch (94) einen Anschlag aufweist, welcher das Auslösemittel (77) in einer Auslöserichtung sperrt, wobei der Anschlag bezüglich der Rastzunge (77) sowie der Rastkante (10) des Grundkörpers (2) derart angeordnet ist, dass die Rastzunge (77) vom Anschlag an der Rastkante (10) festlegbar ist.

2. Abgabevorrichtung (1, 1') gemäss Anspruch 1, **dadurch gekennzeichnet, dass** die Betätigungsvorrichtung (90) derart ausgebildet ist, dass beim Übergang aus der ausgezogenen Stellung in die eingeschobene Stellung des Kolbens (91) das Auslösemittel (77), insbesondere zwingend, auslösbar ist.

3. Abgabevorrichtung (1, 1') gemäss einem der Ansprüche 1 oder 2, **dadurch gekennzeichnet, dass** das Sicherungsmittel derart ausgebildet ist, dass in der ausgezogenen Stellung des Kolbens (91) die Schutzvorrichtung (70), insbesondere zwingend, im ungesicherten Zustand ist.

4. Abgabevorrichtung (1, 1') gemäss einem der Ansprüche 1 bis 3, **dadurch gekennzeichnet, dass** das Sicherungsmittel und/oder das Auslösemittel (77) ein Sperrmittel (78) aufweist, welches derart ausgebildet ist, dass ein Wiederherstellen der Wirkverbindung, insbesondere zwischen Sicherungsmittel und Auslösemittel, des gesicherten Zustand verhindert ist, wenn diese Wirkverbindung einmal gelöst wurde.

5. Abgabevorrichtung (1, 1') gemäss einem der Ansprüche 1 bis 4, **dadurch gekennzeichnet, dass** die Betätigungsvorrichtung (90) eine, insbesondere grundkörperseitig, vorzugsweise an der Fingerplatte (93), ausgebildete, Steuerfläche (95) aufweist, welche beim Übergang aus der ausgezogenen Stellung in die eingeschobene Stellung des Kolbens (91) derart mit der Rastzunge (77), zusammenwirkt, dass die Rastzunge (77) von der Steuerfläche (95) in eine Auslöserichtung bewegbar ist bzw. bewegt wird, wobei eine Verrastung der Rastzunge (77) an der Rastkante (10) des Grundkörpers (2) lösbar ist.

## Claims

1. Dispensing device (1, 1') for dispensing a fluid, in particular for medical use, preferably an injection syringe, comprising:
- a main body (2, 2') with a longitudinal axis (A),
- a receiving chamber (14) formed in the main body (2, 2') for the fluid,
- a dispensing area (4, 4'), which is designed for the arrangement of a cannula (45) communicating with the receiving chamber (14), or in which a cannula (45) communicating with the receiving chamber (14) is arranged, and through which the fluid can be dispensed from the receiving chamber (14),
- wherein the dispensing device (1) comprises a protective device (70) with a rigid protective body (71) arranged on the main body (2), wherein the protective body (71) is designed in such a way and is movable with respect to the main body (2) between a standby position and a protective position in such a way that the cannula (45) arranged or able to be arranged in the dispensing area (4, 4') is completely exposed in the standby position, and the protective body (71), in the protective position, extends completely from the main body (2) over the cannula (45) arranged or able to be arranged in the dispensing area (4, 4'), wherein a trigger means (77) is present which can be triggered directly or indirectly by a user and with which a transfer of the protective body (71) from the standby position to the protective position can be triggered,
wherein the trigger means (77) comprises at least one latching tongue (77) which is formed on the protective body (71) and, in the standby position of the protective body (71), is latched on a latching edge (10) of the main body (2),
wherein a securing means is present with which the protective device (70) can be brought to a secured state and an unsecured state, wherein the protective device (70) in the secured state is secured against a triggering of the transfer from the standby position to the protective position, and, in the unsecured state, the transfer can be triggered,
wherein the securing means is designed in such a way that, in the secured state of the protective device (70), the trigger means (77) is secured by the securing means against being triggered,
wherein the dispensing device (1, 1') has an actuating device (90) with a piston (91) which is movable in the receiving chamber and which is movable from an inserted position to a retracted position in order to introduce the fluid into the receiving chamber (14) and is movable in the opposite direction in order to discharge the fluid,
**characterized in that**
the actuating device (90) is formed as a securing means in such a way that the protective device (70) can be brought to the secured state in the inserted state of the piston (91),
wherein the actuating device (90) has a finger plate (93) for moving the piston (91), which finger plate (93) is connected in particular rigidly to the piston (91) and is designed as a securing means,
wherein the securing means is designed as at least one passage (94) which is formed in the finger plate (93) and through which the at least one latching tongue (77) can protrude when the piston (91) is in the inserted position, wherein the passage (94) has an abutment that blocks the trigger means (77) in a trigger direction, wherein the abutment is arranged with respect to the latching tongue (77), and also to the latching edge (10) of the main body (2), in such a way that the latching tongue (77) can be fixed on the latching edge (10) by the abutment.

2. Dispensing device (1, 1') according to Claim 1, **characterized in that** the actuating device (90) is formed in such a way that, upon the transfer of the piston (91) from the retracted position to the inserted position, the trigger means (77) can be triggered, in particular in a constrained manner.

3. Dispensing device (1, 1') according to either of Claims 1 and 2, **characterized in that** the securing means is formed in such a way that, in the retracted position of the piston (91), the protective device (70) is in the unsecured state, in particular by constraint.

4. Dispensing device (1, 1') according to one of Claims 1 through 3, **characterized in that** the securing means and/or the trigger means (77) has a blocking means (78), which is designed in such a way that a restoration of the operative connection, in particular between securing means and trigger means, of the secured state is prevented once this operative connection has been canceled.

5. Dispensing device (1, 1') according to one of Claims 1 through 4, **characterized in that** the actuating device (90) has a control face (95) which is formed in particular on the main body side, preferably on the finger plate (93), and which, during the transfer of the piston (91) from the retracted position to the inserted position, cooperates with the latching tongue (77), in such a way that the latching tongue (77) is movable or is moved from the control face (95) in a triggering direction, wherein a latching of the latching tongue (77) on the latching edge (10) of the main body (2) can be released.

## Revendications

1. Dispositif de distribution (1, 1') permettant de distribuer un fluide, en particulier pour une application médicale, de préférence seringue d'injection, comprenant:
- un corps de base (2, 2') avec un axe longitudinal (A),
- une chambre de réception (14) pour le fluide formée dans le corps de base (2, 2'),
- une zone de distribution (4, 4'), qui est configurée en vue de l'agencement d'une canule (45) communiquant avec la chambre de réception (14) ou dans laquelle une canule (45) communiquant avec la chambre de réception (14) est disposée, par laquelle le fluide peut être distribué à partir de la chambre de réception (14),
dans lequel le dispositif de distribution (1) comprend un dispositif de protection (70) avec un corps de protection rigide (71) disposé sur le corps de base (2), dans lequel le corps de protection (70) est configuré et est mobile par rapport au corps de base (2) entre une position de disponibilité et une position de protection de telle manière que la canule (45) qui est ou peut être disposée dans la zone de distribution (4, 4') dans la position de disponibilité soit entièrement libérée et que le corps de protection (71) dans la position de protection s'étende à partir du corps de base (2) entièrement au-delà de la canule (45) qui est ou peut être disposée dans la zone de distribution (4, 4'), dans lequel il se trouve un moyen de déclenchement (77) pouvant être déclenché directement ou indirectement par un utilisateur, avec lequel un passage du corps de protection (71) de la position de disponibilité à la position de protection peut être déclenché,
dans lequel le moyen de déclenchement (77) comprend au moins une languette d'encliquetage (77), qui est formée sur le corps de protection (71) et qui se cale dans la position de disponibilité du corps de protection (71) sur une arête d'encliquetage (10) du corps de base (2), dans lequel il se trouve un moyen de blocage, avec lequel le dispositif de protection (70) peut être amené dans un état bloqué et un état non bloqué, dans lequel le dispositif de protection (70) dans l'état bloqué est bloqué contre un déclenchement du passage de la position de disponibilité à la position de protection, et dans l'état non bloqué le passage peut être déclenché,
dans lequel le moyen de blocage est configuré de telle manière que dans l'état bloqué du dispositif de protection (70) le moyen de déclenchement (77) soit bloqué contre un déclenchement par le moyen de blocage, dans lequel le dispositif de distribution (1, 1') présente un dispositif d'actionnement (90) avec un piston (91) mobile dans la chambre de réception, qui est mobile d'une position rentrée à une position étendue pour l'introduction de fluide dans la chambre de réception (14) et dans le sens inverse pour l'en expulser,
**caractérisé en ce que**
le dispositif d'actionnement (90) est configuré comme moyen de blocage de telle manière que le dispositif de protection (70) puisse être amené dans l'état bloqué lorsque le piston (91) est rentré,
dans lequel le dispositif d'actionnement (90) présente une plaque à doigt (93) assemblée en particulier de façon rigide au piston (91) pour le déplacement du piston (91), qui est configurée comme moyen de blocage, dans lequel le moyen de blocage est configuré comme au moins un passage (94) dans la plaque à doigt (93), à travers lequel ladite au moins une languette d'encliquetage (77) peut passer, lorsque le piston (91) se trouve dans la position rentrée, dans lequel le passage (94) présente une butée, qui arrête le moyen de déclenchement (77) dans une direction de déclenchement, dans lequel la butée est disposée par rapport à la languette d'encliquetage (77) ainsi que de l'arête d'encliquetage (10) du corps de base (2) de telle manière que la languette d'encliquetage (77) puisse être bloquée sur l'arête d'encliquetage (10) par la butée.

2. Dispositif de distribution (1, 1') selon la revendication 1, **caractérisé en ce que** le dispositif d'actionnement (90) est configuré de telle manière que lors du passage de la position étendue à la position rentrée du piston (91) le moyen de déclenchement (77) puisse être déclenché, en particulier obligatoirement.

3. Dispositif de distribution (1, 1') selon une des revendications 1 ou 2, **caractérisé en ce que** le moyen de blocage est configuré de telle manière que dans la position étendue du piston (91) le dispositif de protection (70) se trouve dans l'état non bloqué, en particulier obligatoirement.

4. Dispositif de distribution (1, 1') selon l'une quelconque des revendications 1 à 3, **caractérisé en ce que** le moyen de blocage et/ou le moyen de déclenchement (77) présente un moyen d'arrêt (78), qui est configuré de telle manière qu'un rétablissement de la liaison active, en particulier entre le moyen de blocage et le moyen de déclenchement, de l'état bloqué soit empêché, lorsque cette liaison active a été rompue une fois.

5. Dispositif de distribution (1, 1') selon l'une quelconque des revendications 1 à 4, **caractérisé en ce que** le dispositif d'actionnement (90) présente une face de commande (95), en particulier du côté du corps de base, formée de préférence sur la plaque à doigt (93), qui lors du passage de la position étendue à la position rentrée du piston (91) coopère avec la languette d'encliquetage (77) de telle manière que la languette d'encliquetage (77) puisse être ou soit déplacée par la face de commande (95) dans un sens de déclenchement, dans lequel un encliquetage de la languette d'encliquetage (77) sur l'arête d'encliquetage (10) du corps de base (2) peut être libéré.
